# EUROPEAN PATENT APPLICATION

(11) **EP 4 725 977 A1**
(43) Date of publication of application: **15.04.2026**
(21) Application number: 23940124.3
(22) Date of filing: 08.06.2023
(51) Int. Cl.: C08G 63/685, C08G 63/91, C08G 63/60, C08G 63/78, C08G 69/44, C12N 15/87, C12P 7/625, A61K 9/51, A61K 47/34

(54) **EFFICIENT CATIONIC POLYESTER AND USE THEREOF**

(71) Applicant: Integena Inc, Greenbrook, New Jersey 08812-0881 (US)
(72) Inventor: LIU, Gan, Shenzhen, Guangdong 518000 (CN); XIANG, Wenqiang, Shenzhen, Guangdong 518000 (CN); GUAN, Miaomiao, Shenzhen, Guangdong 518000 (CN); DENG, Yang, Shenzhen, Guangdong 518000 (CN)
(74) Representative: Michalski Hüttermann & Partner Patentanwälte mbB
(86) International application number: PCT/CN2023/099194
(87) International publication number: WO 2024/250238

(57) **Abstract**

Disclosed in the present invention are an efficient cationic polyester and the use thereof. The cationic polyester of the present invention is a hyperbranched polymer formed by means of polymerizing three monomers of monomer P, monomer S and monomer M, or two monomers of monomer P and monomer S, with monomer T. The hyperbranched polymer has an end group modified with group E, and the group E has a relative concentration of 0.48-0.75 to group E of a hyperbranched polymer with complete end-group modification. Monomer P is cyclic lactone. Monomer S is an organic acid with an end group containing two or more carboxyl. Monomer M is a compound containing two hydroxyl and one secondary amine or tertiary amine. Monomer T is a compound containing at least three hydroxyl. The end-group modifying compound E which provides group E modification is a compound containing at least one primary amine, secondary amine or tertiary amine group. The cationic polyester of the present invention only has a part of the end groups modified with group E, has higher nucleic acid transfection efficiency when being used for nucleic acid drug delivery, has low cytotoxicity, and can be used in clinical practice.

## Description

### FIELD

The present application relates to the field of nucleic-acid delivery materials, and in particular to a high-efficiency cationic polyester and its applications.

### BACKGROUND

Gene therapy based on nucleic acid delivery is regarded as driving the third industrial transformation in biopharmaceuticals. Compared with other delivery technologies such as DNA or viral vectors, mRNA technology offers higher efficiency, better safety profile, shorter production cycles, and lower manufacturing costs. mRNA technology has achieved a breakthrough in COVID-19 vaccines and is expected to provide new modalities for oncology and immune diseases. However, due to intrinsic instability and low in vivo delivery efficiency of mRNA, broader application has been limited. Effective clinical utility requires appropriate carriers to transport mRNA in vivo. At present, commercially successful delivery technologies are primarily lipid nanoparticles (LNPs) based on ionizable cationic lipids as used by companies such as Moderna and BioNTech. Although LNPs were widely used in COVID-19 vaccines, their relatively modest efficiency and notable side effects substantially restrict their scope of use. Accordingly, the development of new, efficient, and low-toxicity delivery systems remains a major bottleneck for advancement of mRNA technologies.

Yale University previously reported a biodegradable cationic polyester and its preparation method for DNA and mRNA delivery . Using in vitro synthetic biology, a linear polymer obtained by immobilized lipase-catalyzed polymerization of three monomers (P, S, M), is used directly or terminal-group modified to give a biodegradable cationic polyester, PACA-E, for DNA/mRNA delivery. Compared with commercial reagents, the polyester exhibited higher transfection efficiency and lower toxicity. Nevertheless, its transfection efficiency is still insufficient, and solvation requires toxic organic solvents, limiting its practical clinical use.

Building on P/S/M polymerization, BaiDa Liankang Biotechnology (Shenzhen) Co., Ltd. innovatively introduced a new monomer T-or replaced M with T-to prepare a novel hyperbranched cationic polyester, HBPA-E (Chinese Patent Application No. 202211419916.1). Relative to PACA-E, HBPA-E shows higher transfection efficiency and lower cytotoxicity, satisfying clinical needs better.

However, further study revealed that the transfection efficiency of HBPA-E still has room for improvement, which is of significant value for clinical application.

### SUMMARY

An object of the present application is to provide an improved, high-efficiency cationic polyester and its applications.

To achieve the foregoing, the following technical solution is provided:

In one aspect, the present application discloses a cationic polyester which is a hyperbranched polymer formed by copolymerizing monomer P, monomer S, and monomer M, or monomer P and monomer S with monomer T; terminal groups of the hyperbranched polymer are modified with group E; and the relative concentration of terminal group E is 0.48-0.75 with respect to the terminal group E of a completely end-group-modified hyperbranched polymer. Monomer P is a lactone; monomer S is an organic acid with an end group containing two or more carboxyl groups; monomer M is a compound containing two hydroxyl groups and a secondary or tertiary amine; monomer T is a compound containing three or more hydroxyl groups; and the end-group modifying compound E providing group E is a compound containing at least one primary, secondary, or tertiary amine.

It should be noted that, based on HBPA-E disclosed in patent application 202211419916.1, the present application has, through in-depth study, found that by adjusting the amount of the end-group modifying compound E, the terminal groups of the hyperbranched polymer can be only partially modified with group E, that is, the relative concentration of terminal group E is 0.48-0.75 with respect to the terminal group E of a completely end-group-modified hyperbranched polymer. Compared with HBPA-E in patent application 202211419916.1, in which all terminal groups are modified with group E, the HBPA-E having this specific proportion of end-group modification in the present application shows higher transfection efficiency, i.e., the so-called high-efficiency cationic polyester of this application; for example, in one embodiment of the present application, HBPA-E with this specific proportion of end-group modification can increase transfection efficiency by 1.5-3 times.

A "completely end-group-modified" hyperbranched polymer refers to, for example, the case in patent application 202211419916.1, where a large excess of end-group modifying compound E is used to modify the end groups of the hyperbranched polymer, so that all terminal groups are modified with group E, i.e., a fully end-group-modified hyperbranched polymer. In other words, a completely end-group-modified hyperbranched polymer is one in which all terminal groups are bearing group E. A relative concentration of terminal group E of 0.48-0.75, with respect to the terminal group E of a completely end-group-modified hyperbranched polymer, can be understood to mean that 48%-75% of all terminal groups on a given hyperbranched polymer molecule are modified with group E.

It is considered that, for the HBPA-E of the present application in which only part of the terminal groups are modified, the nanoparticles formed with mRNA differ markedly from fully modified HBPA-E in the amount and spatial distribution of surface charge; accordingly, serum stability, cellular uptake, and endosomal escape efficiency can be improved.

It should also be noted that the core of the present application is the finding that HBPA-E with a specific proportion of E-modified terminal groups exhibits higher transfection efficiency; on this basis, HBPA-E with this specific proportion of end-group modification also retains the same nucleic-acid delivery performance and advantages as the HBPA-E disclosed in patent application 202211419916.1, such as low cytotoxicity and better suitability for clinical use.

Further, the preparation of HBPA-E with this specific proportion of terminal-group modification can follow the method in patent application 202211419916.1, with adjustment of the amounts of the coupling agent and the end-group modifying compound E, i.e., reducing their equivalents relative to the hyperbranched polymer so that only a portion of the polymer termini are modified with group E. For example, in one embodiment of the present application, adding only 4-7 equivalents of the end-group modifying compound E relative to the hyperbranched polymer affords a cationic polyester in which only part of the terminal groups are modified with group E and the relative concentration of terminal group E is 0.48-0.75.

In one embodiment of the present application, monomer P is a lactone having an aliphatic chain length of 6 to 35.

In one embodiment of the present application, monomer P is selected from, but not limited to, at least one of caprolactone, dodecalactone, pentadecalactone, and hexadecalactone.

In one embodiment of the present application, monomer S is an organic acid having a carbon chain length of 3 to 18.

In one embodiment of the present application, monomer S is selected from, but not limited to, at least one of adipic acid, sebacic acid, and 1,2,3-propanetricarboxylic acid.

In one embodiment of the present application, monomer M is a compound having a carbon chain length of 4 to 36 and containing two hydroxyl groups and one secondary or tertiary amine.

In one embodiment of the present application, monomer M is selected from, but not limited to, at least one of diethanolamine, methyldiethanolamine, and ethyldiethanolamine.

In one embodiment of the present application, monomer T is a compound having a carbon chain length of 4 to 54 and containing three or more hydroxyl groups.

In one embodiment of the present application, monomer T is selected from, but not limited to, at least one of trimethylolpropane, 3-(hydroxymethyl)-1,5-pentanediol, triethanolamine, and N,N,N',N'-tetrakis(2-hydroxyethyl)ethylenediamine.

In one embodiment of the present application, the end-group modifying compound E that provides group E is at least one selected from E1 to E26.

Preferably, the end-group modifying compound E that provides group E is at least one selected from E1-E10, E12, E14-E21, E25, and E26.

More preferably, the end-group modifying compound E that provides group E is at least one selected from E1, E2, E3, E4, E5, E6, E9, E10, E12, E14, E15, E25, and E26.

It should be noted that the key of the present application lies in the finding that HBPA-E with a specific proportion of end-group modification exhibits higher transfection efficiency; it can be understood that patent application 202211419916.1 discloses that end-group modifying compounds E1-E26 can all be used for the hyperbranched polymer, and, under the same inventive concept as the present application, when only a specific proportion of end groups is modified, the transfection efficiency can likewise be improved to varying degrees.

In one embodiment of the present application, the cationic polyester is a hyperbranched polymer of "Formula I." 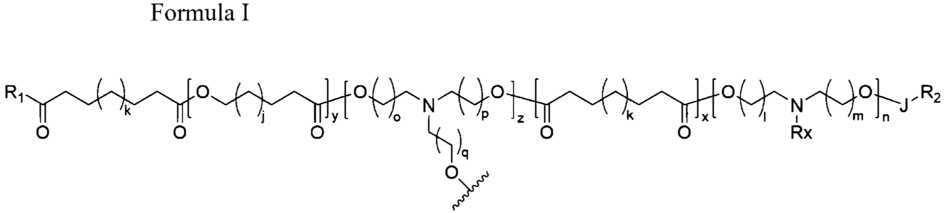
Where x, y, z are independent integers from 1 to 200,
n is an integer from 0 to 200,
j and k are integers from 0 to 30,
l, m, o, p, and q are independent integers from 1 to 20,
Rₓ is hydrogen; a substituted or unsubstituted C1-C18 alkyl; a substituted or unsubstituted aryl with at least one phenyl ring; a substituted or unsubstituted heterocycle with at least one hetero ring; or a substituted or unsubstituted C1-C18 alkoxy having at least one oxygen.

R₁ is fully or partially modified by group E provided by the end-group modifier E, with any remainder being hydroxyl.

J is hydrogen or carbonyl; when J is hydrogen, no R₂ is present; when J is carbonyl, R₂ is group E provided by the end-group modifier E.

In Formula I, cut lines indicate branching; the first monomer connected at a branch is P or S, and subsequent monomers form the hyperbranched structure.

"Subsequent monomers" refers to monomers further connected to the branch per Formula I such that higher-order branching occurs iteratively, forming a dendritic hyperbranched architecture. Within a given polymer, some J may be hydrogen and others carbonyl; where J is hydrogen, that end group is not modified with group E, i.e., only a portion of end groups bear group E.

It should be noted that R₁ and/or R₂ in the present application are modified with groups E provided by the end-group modifying compound E. During end-group modification of the hyperbranched polymer with the end-group modifying compound E, the compound E loses one hydrogen atom to form group E, which then becomes bound to the terminal of the hyperbranched polymer. When N,N'-carbonyldiimidazole (CDI) is used as a coupling agent, end-group modification with group E occurs at both terminal positions of the hyperbranched polymer of Formula I. In this case, in order to connect group E to the hyperbranched polymer, CDI provides a carbonyl group at the R₂ terminal position to link group E and the hyperbranched polymer, while at the R₁ position the end-group modifying compound E likewise loses one hydrogen to form group E, which then directly bonds to the hyperbranched polymer.

Furthermore, when the hyperbranched polymer is formed only from monomers P, S, and T (i.e., n = 0), and J is a carbonyl group, one end of the carbonyl is connected to R₂ and the other end is connected to the hydroxyl of monomer T, thereby forming an E-modified terminal group. It can be understood that, in this case, the general structure of Formula I is correspondingly adapted such that monomer T is located at the terminal and is linked to the carbonyl J.

In one embodiment of the present application, the number-average molecular weight of the cationic polyester is 1-30 kDa, preferably 2-20 kDa.

In another aspect, the present application discloses the use of the cationic polyester in nucleic-acid drug delivery.

In one embodiment of the present application, the nucleic-acid drug includes, but is not limited to, mRNA (messenger RNA), circRNA (circular RNA), saRNA (self-amplifying RNA), siRNA (small interfering RNA), miRNA (microRNA), antisense oligonucleotides (ASO), shRNA (small hairpin RNA), small activating RNA, and DNA.

It should be noted that the key of the present application lies in the finding that HBPA-E with a specific proportion of end-group modification exhibits higher transfection efficiency and has the advantage of low cytotoxicity. It will be understood that, due to their low cytotoxicity, the cationic polyesters of the present application can be used not only for nucleic-acid delivery, but also for other similar operations in which substances need to be delivered into cells.

In one embodiment of the present application, nucleic-acid drug delivery comprises encapsulating the nucleic-acid drug with the cationic polyester of the present application and delivering it into cells.

In one embodiment of the present application, the cell types include, but are not limited to, HEK293T, A549, HeLa, U87, HUVEC, Jurkat, RAW264.7, iPSC, and MSC.

In a further aspect, the present application discloses a nucleic-acid delivery particle comprising an encapsulating material and a nucleic acid encapsulated within the encapsulating material; the encapsulating material is the cationic polyester of the present application or contains at least a portion of the cationic polyester of the present application; the nucleic acid is at least one selected from mRNA (messenger RNA), circRNA (circular RNA), saRNA (self-amplifying RNA), siRNA (small interfering RNA), miRNA (microRNA), antisense oligonucleotides (ASO), shRNA (small hairpin RNA), small activating RNA, and DNA.

It should be noted that, because the nucleic-acid delivery particles of the present application employ the cationic polyester disclosed herein, they not only can enhance transfection efficiency, but also exhibit relatively low cytotoxicity, thereby offering improved prospects for clinical use. It will be understood that the key of the present nucleic-acid delivery particles lies in the use of the cationic polyester of this application, while the specific encapsulation methods and nucleic-acid delivery methods may follow the prior art.

It should also be noted that the encapsulating material of the present application may, depending on the design or needs of the encapsulating layer, further include materials other than the present cationic polyester, which are not specifically limited herein. It will be understood that, so long as the encapsulating material contains the cationic polyester of the present application, transfection efficiency can be improved and cytotoxicity reduced to some extent.

In one embodiment of the present application, the nucleic-acid delivery particles have a particle size of 30-500 nm.

In a further aspect, the present application discloses a kit for nucleic-acid drug delivery comprising at least one of the following components:
(a) the cationic polyester of the present application;
(b) the nucleic-acid delivery particles of the present application.

It should be understood that the nucleic-acid drug delivery kit of the present application may be a kit in which a specific nucleic-acid drug is already encapsulated, or may comprise the cationic polyester of the present application, with the user selecting and encapsulating the desired nucleic-acid drug as needed. The key feature is that the kit contains the cationic polyester and/or the nucleic-acid delivery particles of the present application; other conventional reagents required for nucleic-acid delivery, such as certain lipid or polymer materials, may be obtained according to the prior art or from commercial sources. For convenience of use, some of these reagents may also be included in the kit of the present application, which is not further limited herein.

In a further aspect, the present application discloses a method for improving transfection efficiency in nucleic-acid drug delivery, comprising encapsulating a nucleic-acid drug with the cationic polyester of the present application, or with an encapsulating material containing the cationic polyester of the present application, to form nucleic-acid delivery particles, and using the nucleic-acid delivery particles to transfect cells with the nucleic-acid drug.

It should be understood that the key to the method of the present application lies in the use of the cationic polyester of the present application to improve transfection efficiency. In one embodiment of the present application, the mRNA delivery efficiency of the cationic polyester with a specific proportion of end-group modification as described herein can be up to three times that of a cationic polyester with fully modified end groups, while likewise exhibiting low cytotoxicity. In addition, the cationic polyester of the present application is soluble in ethanol, which is more favorable for clinical use and confers substantial practical application potential. It will be appreciated that, in the method for improving transfection efficiency of the present application, the critical factor is the use of the cationic polyester of the present application; other cell-transfection operations and reagents may follow the prior art and are not particularly limited herein.

It should further be noted that, in the method for improving transfection efficiency of the present application, the cationic polyester of the present application may be used directly as the encapsulating material, or may be added to an existing encapsulating material; it will be understood that, so long as the encapsulating system contains the cationic polyester of the present application, transfection efficiency can be improved to varying degrees.

In another aspect, the present application discloses the use of the cationic polyester of the present application, or the nucleic-acid delivery particles of the present application, or the kit of the present application, in the treatment of diseases based on nucleic-acid drug delivery.

In a further aspect, the present application discloses a method for administering a nucleic-acid drug in vivo, including encapsulating the nucleic-acid drug with the cationic polyester of the present application, or with an encapsulating material containing the cationic polyester of the present application, to form nucleic-acid delivery particles and delivering the nucleic-acid drug by means of the nucleic-acid delivery particles; or directly using the nucleic-acid delivery particles of the present application to deliver the nucleic-acid drug.

It should be understood that the key to the administration method of the present application lies in the use of the cationic polyester or the nucleic-acid delivery particles for nucleic-acid drug delivery; the specific procedures for in vivo delivery and any auxiliary materials required may be selected according to the prior art and are not particularly limited herein.

By virtue of the foregoing technical solution, the beneficial effects of the present application are as follows:

The cationic polyester of the present application has a relative concentration of terminal group E of 0.48-0.75 with respect to the terminal group E of a completely end-group-modified hyperbranched polymer, i.e., only a portion of the terminal groups are modified with group E, thereby further improving transfection efficiency while also maintaining low cytotoxicity and meeting clinical-use requirements.

### BRIEF DESCRIPTION OF THE DRAWINGS

FIG. 1 is a schematic diagram of the synthetic route for the hyperbranched polymer and its end-group E modification in the embodiments of the present application.
FIG. 2 is the spectrum of HBPA-E14-3 before and after end-group modification in the embodiments of the present application.
FIG. 3 is the GPC chromatogram of HBPA-E14-1 and PACA-E14 in the embodiments of the present application.
FIG. 4 is a quantitative plot of terminal E14 modification for HBPA-E14-1 and PACA-E14 in the embodiments of the present application.
FIG. 5 shows the particle-size measurement results of complexes of HBPA-E14-2 with mRNA in the embodiments of the present application.
FIG. 6 shows the mRNA transfection efficiency of different HBPA-E materials in A549 and HEK 293T cells in the embodiments of the present application.
FIG. 7 shows the mRNA transfection efficiency of HBPA-E14-3 modified by two different methods (CDI and DIC) at various polymer/mRNA mass ratios in the embodiments of the present application.
FIG. 8 shows the DNA transfection efficiency of different HBPA-E materials in HEK 293T cells in the embodiments of the present application.
FIG. 9 shows the mRNA transfection efficiency of HBPA-E polymers bearing 26 different terminal groups E in A549 cells in the embodiments of the present application.
FIG. 10 shows the cytotoxicity results for complexes of the polymers and mRNA in the A549 cell model in the embodiments of the present application.
FIG. 11 shows the in-vivo mRNA transfection performance of HBPA-E in the embodiments of the present application.
FIG. 12 shows spectra of HBPA-E after E14 end-group modification at different degrees of substitution in the embodiments of the present application.
FIG. 13 is a plot of the relative concentration of E14 at different degrees of end-group modification in the embodiments of the present application.
FIG. 14 shows the mRNA transfection efficiency in A549 cells of HBPA-E with different degrees of E14 end-group modification in the embodiments of the present application.
FIG. 15 shows the mRNA transfection efficiency in A549 cells of HBPA-E with different degrees of E2 end-group modification in the embodiments of the present application.
FIG. 16 shows the in-vivo mRNA transfection performance of HBPA-E with different degrees of E14 end-group modification in the embodiments of the present application.

### DETAILED DESCRIPTION

Patent application 202211419916.1 discloses using 40 equivalents of the terminal-group modifying compound E to prepare HBPA-E, i.e., a large excess of the terminal-group modifying compound E is added in the production process to achieve the maximum degree of terminal-group modification of the hyperbranched polymer, such that essentially all terminal groups of the hyperbranched polymer are modified with group E, namely a fully terminal-group-modified hyperbranched polymer.

However, in the course of research and practice in the present application, it was found that when only a portion of the terminal groups of the hyperbranched polymer are modified with group E, the resulting HBPA-E exhibits higher transfection efficiency than HBPA-E in which all terminal groups are modified with group E, particularly when the relative concentration of terminal group E is 0.48-0.75 with respect to the terminal group E of a fully terminal-group-modified hyperbranched polymer, in which case the improvement in transfection efficiency is more pronounced.

The present application will be further described in detail below with reference to specific embodiments. The following embodiments serve only to further illustrate the present application and should not be construed as limiting the scope of the present application.

Unless otherwise specified, the technical means used in the embodiments are conventional means well known to those skilled in the art.

### Example I

### I. Synthesis of Hyperbranched Polymer (HBPA) and Terminal-group E-Modified HBPA (HBPA-E)

### (1) HBPA Synthesis

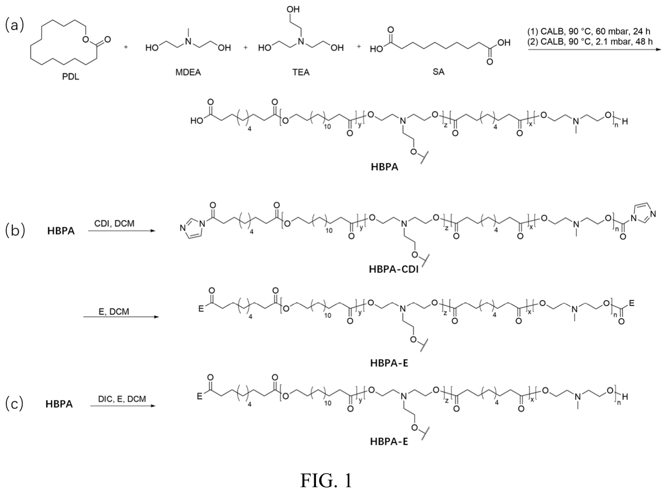

In this example, a hyperbranched polymer (HBPA) was first prepared by copolymerizing four monomers P, S, M, and T, and then the terminal groups of HBPA were modified with group E to obtain HBPA-E. Specifically, monomer P was pentadecalactone (PDL), monomer S was sebacic acid (SA), monomer M was methyldiethanolamine (MDEA), and monomer T was triethanolamine (TEA). The synthetic route for HBPA is shown in FIG. 1(a). Seven experimental runs and two controls were conducted. The four monomers P (PDL) / S (SA) / M (MDEA) / T (TEA) were charged into a round-bottom flask according to the feed molar ratios listed in Table 1, and immobilized lipase (CALB) was added at 10 wt% relative to the total mass of the monomers, followed by diphenyl ether at 200 wt% relative to the total mass of the monomers.

After purging the reaction system with argon three times, the mixture was stirred and heated at 90 °C under 60 mbar for 24 hours, and then further reacted at 2.1 mbar for 48 hours. After the reaction was complete, the mixture was filtered to remove the lipase. N-hexane was added to the filtrate, followed by vortexing and centrifugation, and the supernatant was decanted. The precipitate was dissolved in dichloromethane, then re-precipitated by adding n-hexane, centrifuged, and the supernatant was discarded. The above procedure was repeated three times. The final precipitate was dried under vacuum for one day to give HBPA. Among these, the two control polymers synthesized were linear polymers PACA.

**Table 1. Characterization of polymers from different feed ratios and E14 modification.**

| Serial No. | Polymer name | P/S/M/T (monomer feed molar ratio) | P/S/M/T (repeat-unit molar ratio)^{a} | Mn^{b} | PDI^{b} | Terminal-group modification method | Solubility in ethanol (mg/mL) |
|---|---|---|---|---|---|---|---|
| Con 1 | PACA-E14 | 1: 9: 9: 0 | 1:10: 9.11: 0 | 8769 | 1.97 | CDI | <10 |
| Con 2 | PACA-E14-DIC | 1: 9: 9: 0 | 1: 9.8: 9.05: 0 | 8665 | 1.92 | DIC | <10 |
| Exp 1 | HBPA-E14-1 | 1:9:7.7:0.9 | 1:9:6.3:1.0 | 8296 | 4.12 | CDI | >25 |
| Exp 2 | HBPA-E14-2 | 1: 9: 6.2: 1 | 1: 9.9: 4.9: 1.0 | 5457 | 3.33 | CDI | >25 |
| Exp 3 | HBPA-E14-3 | 1: 9: 6: 2 | 1: 9.0: 4.5: 1.9 | 6548 | 3.22 | CDI | >25 |
| Exp 4 | HBPA-E14-3-DIC | 1: 9: 6: 2 | 1: 8.9: 4.4: 1.8 | 6643 | 3.26 | DIC | >25 |
| Exp 5 | HBPA-E14-4 | 1: 9: 4.5: 3 | 1: 8.9: 4.1: 2.9 | 7534 | 2.85 | CDI | >25 |
| Exp 6 | HBPA-E14-5 | 1: 9: 3: 4 | 1: 8.5: 2.9: 3.6 | 7473 | 2.95 | CDI | >25 |
| Exp 7 | HBPA-E14-6 | 1: 9: 1.5: 5 | 1: 8.7: 1.2: 4.7 | 7362 | 3.15 | CDI | >25 |

In Table 1, "a" is calculated from 1H-NMR and "b" is calculated from GPC; "Con" denotes Control and "Exp" denotes Experiment.

### (2) Terminal-group E modification

In this example, as shown in Table 1, terminal-group modifier E14 was used, and two different terminal-group modification methods were employed, using N,N'-carbonyldiimidazole (CDI) or diisopropylcarbodiimide (DIC) as the coupling agent to modify the polymer terminal groups.

### Modification Method 1 (CDI as coupling agent):

As shown in FIG. 1(b), 250 mg of HBPA was dissolved in 5 mL of anhydrous dichloromethane (DCM), and 40 equivalents of CDI were added under stirring. The reaction system was purged with argon three times and then stirred at room temperature overnight. The reaction mixture was concentrated to about 3 mL, three volumes of diethyl ether were added, the mixture was vortexed and centrifuged, and the precipitate was discarded. The supernatant was rotary evaporated to dryness under reduced pressure, redissolved in anhydrous DCM, and 40 equivalents of E14 were added under stirring. The reaction was continued at room temperature for 24 hours. After completion, an equal volume of deionized water was added to the reaction mixture, vortexed and centrifuged to separate the phases, and the upper aqueous phase was removed. An equal volume of deionized water was added again, and this washing procedure was repeated five times. Three volumes of n-hexane were then added to the lower dichloromethane solution, followed by vortexing and centrifugation. The resulting precipitate was dried under vacuum for one day to obtain HBPA-E14. For Control 1, the corresponding product obtained was PACA-E14.

### Modification Method 2 (DIC as coupling agent):

As shown in FIG. 1(c), 250 mg of HBPA was dissolved in 5 mL of anhydrous DCM, and 40 equivalents of DIC and 40 equivalents of E14 were added under stirring. The reaction system was purged with argon three times and then stirred at room temperature overnight. After completion, an equal volume of deionized water was added to the reaction mixture, which was vortexed and centrifuged, and the upper aqueous phase was removed. This washing step with an equal volume of deionized water was repeated three times. Three volumes of n-hexane were then added to the lower dichloromethane phase, followed by vortexing and centrifugation. The resulting precipitate was washed with a small amount of ethanol and then dried under vacuum for one day to obtain HBPA-E14, for example, HBPA-E14-3-DIC was prepared in Experiment 4. For Control 2, the corresponding product obtained was PACA-E14-DIC.

Using a method similar to that of Experiment 3, 25 additional terminal-group modifiers E1-E26 (excluding E14) were further used to modify the terminal groups of HBPA, thereby synthesizing other HBPA-E polymers.

The 26 terminal-group modifier E1-E26 are as follows:

| | | | | | |
|---|---|---|---|---|---|
| E1 | 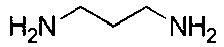 | E10 | 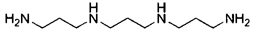 | E18 | 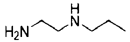 |
| E2 | 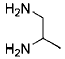 | E11 | 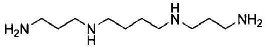 | E19 | 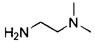 |
| E3 | 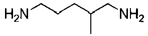 | E12 | 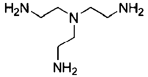 | E20 | 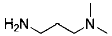 |
| E4 | 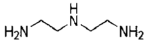 | | | E21 | 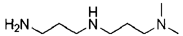 |
| E5 | 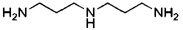 | E13 | 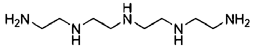 | E22 | 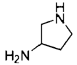 |
| E6 | 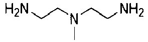 | E14 | 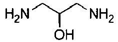 | E23 | 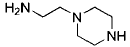 |
| E7 | 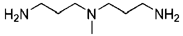 | E15 | 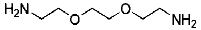 | E24 | 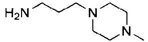 |
| E8 | 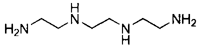 | E16 | 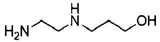 | E25 | 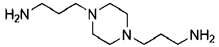 |
| E9 | 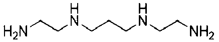 | E17 | 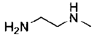 | E26 | 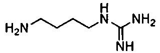 |

### II. Structural Characterization of HBPA and HBPA-E

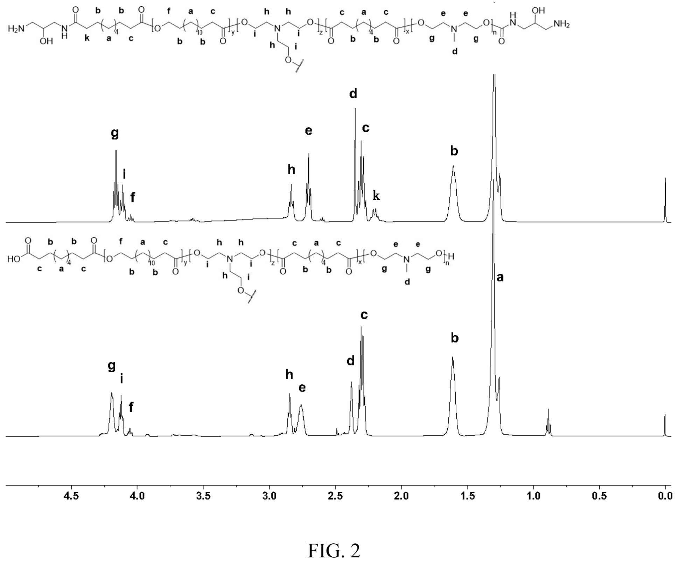

The molecular structures of HBPA and HBPA-E were characterized by 1H-NMR. Results are summarized in Table 1, with representative spectra shown in FIG. 2. The 1H-NMR spectra (solvent: CDCl₃) of the HBPA from Experiment 2 before (HBPA-3) and after (HBPA-E14-3) terminal-group modification are shown in FIG. 2. In the spectra, the characteristic peaks of the PDL unit appear at f (δ ≈ 4.08 ppm), the SA unit at b (δ ≈ 1.60 ppm), the MDEA unit at g (δ ≈ 4.20 ppm), and the TEA unit at h (δ ≈ 2.85 ppm). The presence of TEA signals indicates that the polymer structure matches the expectation and suggests a hyperbranched architecture, while the appearance of peak k (δ ≈ 2.22 ppm) confirms successful terminal-group modification with group E. By integrating the characteristic peaks, the ratios of the repeat units were calculated. For HBPA-E14-3 prepared via CDI coupling, the P/S/M/T molar ratio was determined to be 1:9:4.5:1.9, which is close to the feed ratio of 1:9:6:2. This indicates that the monomers reacted essentially to completion and that the reaction efficiency was high. Additional 1H-NMR data are provided in Table 1. The results further show that even as the feed amount of T increased, the enzyme-catalyzed reaction proceeded nearly to completion and afforded hyperbranched polymers in a highly controllable manner without crosslinking.

The experiments showed that reducing the amount of monomer M had little impact on the formation of the hyperbranched polymer. Accordingly, we further examined the case where the proportion of monomer M was reduced to zero, i.e., the polymerization was carried out using only monomers P, S, and T. Specifically, the feed molar ratio was P:S:T = 1:9:6, and all other steps and parameters were the same as in "(1) HBPA Synthesis." The results indicated that polymerization of P, S, and T likewise afforded a polymer with a hyperbranched structure without crosslinking. The hyperbranched polymer obtained from P/S/T exhibited physicochemical properties similar to those of the hyperbranched polymer obtained from P/S/M/T.

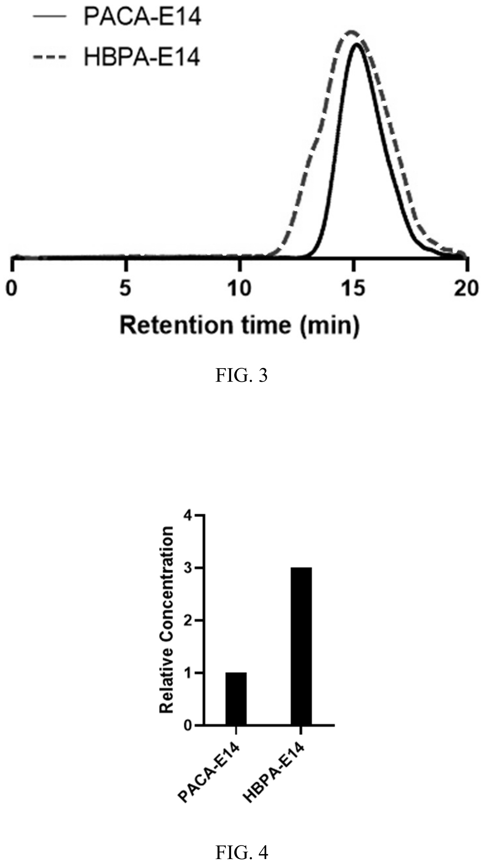

Meanwhile, the molecular weight and distribution of HBPA-E were characterized by gel permeation chromatography (GPC). GPC measurements were conducted at 35 °C using a Waters 1515 system equipped with a 2414 refractive index (RI) detector. The mobile phase was DMF containing 0.1% LiBr at a flow rate of 1 mL/min, and linear poly(methyl methacrylate) standards were used for calibration. Results are listed in Table 1, with representative traces shown in FIG. 3. FIG. 3 displays the GPC chromatograms of HBPA-E14-1 from Experiment 3 and the PACA-E14 from Control 1. Both the hyperbranched HBPA-E14-1 and the linear PACA-E14 exhibited single peaks, with number-average molecular weights (Mn) of 8,296 and 8,769 Da, respectively, and PDIs of 4.12 and 1.97, respectively. Although the Mn values were similar, the PDIs differed markedly, indicating a significant structural difference between HBPA-E14-1 and PACA-E14 that is attributable to the branching in HBPA. PDIs for the other HBPA-E14 samples (as shown in Table 1) were similar to that of HBPA-E14-1.

In addition, HBPA-E14 and PACA-E14 prepared as in Table 1 were dissolved in ethanol to assess solubility. As shown in Table 1, the branched HBPA-E samples readily dissolved in ethanol at concentrations > 25 mg/mL, whereas the linear PACA-E samples were poorly soluble in ethanol, with solubilities < 10 mg/mL.

Finally, the content of terminal E14 groups was quantified for HBPA-E14 (Mn = 8,296) and PACA-E14 (Mn = 8,769) with similar number-average molecular weights using the ninhydrin colorimetric assay. Specifically, (1) Prepare the ninhydrin reagent by dissolving 200 mg ninhydrin in 9 mL ethanol and bringing to 10 mL with ethanol to obtain a 20 mg/mL ethanolic solution. (2) For the assay, about 10 mg of each polymer sample (HBPA-E14 and PACA-E14) was dissolved to 5 mg/mL in ethanol. Then 100 µL of each sample solution was mixed with 200 µL of the ninhydrin solution, shaken at 60 °C for 10 min, cooled to room temperature, and 200 µL aliquots were taken to measure absorbance at 570 nm. The absorbance values were converted to relative terminal E14 concentrations. As shown in FIG. 4, setting the terminal E14 concentration of PACA-E14 to 1, the relative terminal E14 concentration of HBPA-E14-1 at the same material concentration was 3.01, indicating that HBPA-E14-1 contains roughly three times the amount of terminal E14 as PACA-E14. Given the very similar molecular weights, the significantly higher terminal-group content in HBPA-E14-1 is attributed to its branched architecture, further corroborating the hyperbranched structure of HBPA.

It will be understood that, in addition to pentadecalactone (PDL), other conventional lactones can also be used as monomer P in this example to prepare hyperbranched polymers with a highly branched structure-e.g., lactones with C6-C35 aliphatic chains, including but not limited to caprolactone, dodecalactone, and hexadecalactone. For monomer S, besides sebacic acid (SA), other conventional organic acids bearing two or more terminal carboxyl groups-e.g., C3-C18 organic acids, including but not limited to adipic acid and 1,2,3-propanetricarboxylic acid-are also suitable for this example and yield hyperbranched polymers. For monomer M, in addition to methyldiethanolamine (MDEA), other conventional compounds bearing two hydroxyl groups and a secondary or tertiary amine-e.g., C4-C36 compounds, including but not limited to diethanolamine and ethyldiethanolamine-are likewise applicable to this example and give hyperbranched polymers. For monomer T, besides triethanolamine (TEA), other conventional polyols containing three or more hydroxyl groups-e.g., C4-C54 polyols, including but not limited to trimethylolpropane, 3-(hydroxymethyl)-1,5-pentanediol, and N,N,N',N'-tetrakis(2-hydroxyethyl)ethylenediamine-are applicable to this example and can also be used to prepare hyperbranched polymers.

Therefore, HBPA-E in this example may adopt the hyperbranched structure of Formula I:

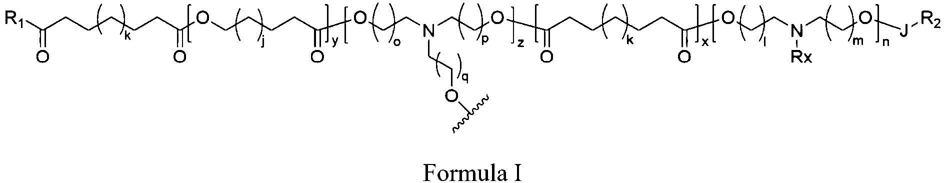

Here, x, y, and z are independent integers from 1 to 200.
n is an integer from 0 to 200.
j and k are integers from 0 to 30.
1, m, o, p, and q are independent integers from 1 to 20.

Rₓ is hydrogen; or a substituted or unsubstituted C1-C18 alkyl; or a substituted or unsubstituted aryl containing at least one phenyl ring; or a substituted or unsubstituted heterocycle containing at least one hetero ring; or a substituted or unsubstituted C1-C18 alkoxy having at least one oxygen atom.

R₁ is either fully modified with group E provided by the terminal-group modifier E, or partially modified with group E with the remainder being hydroxyl groups.

J is hydrogen or a carbonyl; when J is hydrogen, there is no R₂; when J is carbonyl, R₂ is group E provided by the terminal-group modifier E.

In Formula I, the cut lines indicate branching; the first monomer connected at a branch point is P or S, and subsequent monomers are added to form a hyperbranched structure.

The number-average molecular weight (Mn) of HBPA-E in this example can be tailored by adjusting production conditions to 1-30 kDa, which is suitable for general nucleic acid delivery needs; preferably, HBPA-E with Mn of 2-20 kDa provides superior nucleic acid delivery performance.

### III. Characterization of Materials and mRNA Complexes

After preparing the polymer HBPA-E14-2, it was mixed with Firefly luciferase mRNA (N1-Me-Pseudo UTP; Nanjing Vazyme Biotech Co., Ltd.) at various mass ratios, and the hydrodynamic diameter of the resulting complexes was measured. Complexes of PACA-E14 with mRNA were prepared in parallel as controls. Specifically, HBPA-E14-2 and PACA-E14 were dissolved in ethanol and DMSO, respectively, to 25 mg/mL. Aliquots of the polymer solutions were then added to a fixed volume of pH 4.9 sodium acetate (NaAc) buffer with vortexing. In parallel, an mRNA solution (20 µg/mL) was prepared in pH 4.9 NaAc buffer. Equal volumes of the polymer solution were added to the mRNA solution and vortex-mixed to give polymer/mRNA mass ratios of 25:1, 50:1, 75:1, 100:1, 150:1, and 200:1. Both HBPA-E14-2 and PACA-E14 were tested at each of the above mass ratios. Polymer amounts were calculated according to the target ratio; for example, for a 25:1 ratio, 20 µL of the 25 mg/mL polymer stock (i.e., 500 µg polymer) was added to 980 µL of pH 4.9 NaAc buffer with vortexing. An equal volume of the mRNA solution (20 µg/mL, pH 4.9 NaAc) was then added and vortex-mixed, yielding a final polymer/mRNA mass ratio of 25:1.

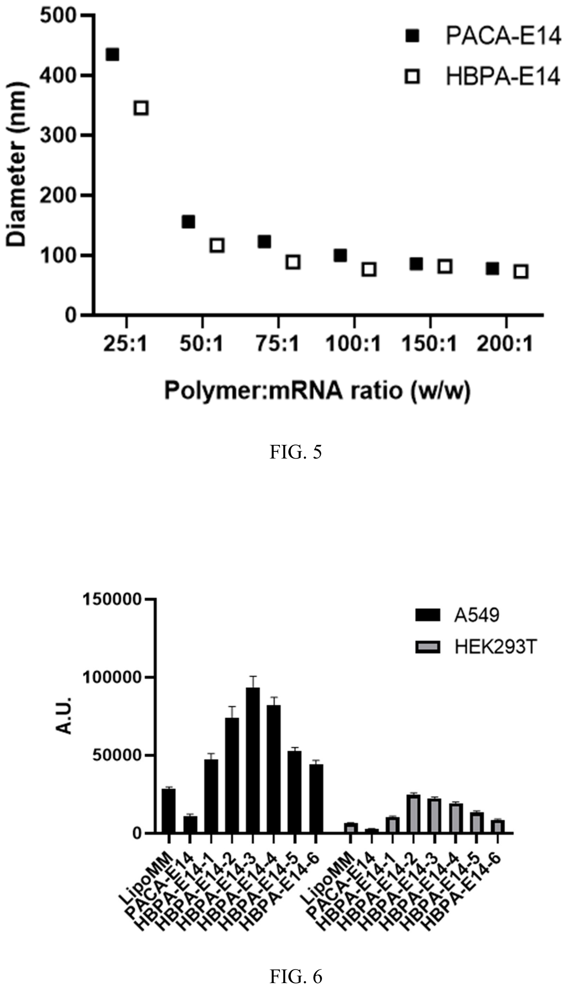

Complex sizes were measured at 25 °C using a particle-size analyzer (Malvern Panalytical Zetasizer Pro). As shown in FIG. 5, the x-axis denotes the polymer/mRNA mass ratio and the y-axis denotes the complex diameter (nm).

The results in FIG. 5 show that HBPA-E14-2/mRNA complexes have diameters exceeding 300 nm at a 25:1 mass ratio, whereas at 50:1-200:1 the diameters drop markedly and stabilize in the 100-200 nm range. This indicates that complex formation between HBPA-E14-2 and mRNA becomes stable at 50:1 ratio or above. The PACA-E14/mRNA complexes exhibited a similar trend.

It is understood that, as needed, the polymer/nucleic acid mass ratio can be adjusted to obtain delivery particles with diameters of 30-500 nm; particles within this size range generally meet typical requirements for nucleic acid delivery. Particles of larger or smaller size can also be prepared as required.

### IV. In Vitro mRNA and DNA Transfection Efficiency of HBPA-E

After preparing complexes of HBPA-E with luciferase-expressing mRNA or DNA (Firefly Luciferase DNA, Guangzhou Aiji Biotechnology Co., Ltd.), transfection efficiencies were evaluated across different cell models in this example. Transfection procedure: A549 and HEK293T cells (ATCC) were seeded in 48-well plates (2.5 × 10⁴ cells/well). After 12 h of adhesion in DMEM at 37 °C with 5% CO₂, the medium was replaced (250 µL/well). Test samples and positive controls were added and incubated for 24 h (mRNA) or 48 h (DNA), with dosing targeted a final 2 µg/mL mRNA per well. The method for preparing complexes of the test material with mRNA or DNA is the same as above ("III. Characterization of Materials and mRNA Complexes"). Positive controls are Lipofectamine MessengerMAX (LipoMM) for mRNA and Lipofectamine 2000 (Lipo2k) for DNA.

Cell lysis and luminescence readout: At 24 h (mRNA) or 48 h (DNA) post-transfection, the culture medium was aspirated and plates were placed at -80 °C for 10 min, then, plates were equilibrated at 4 °C. 40 µL lysis buffer was added per well and incubated for 5 min, followed by 280 µL assay buffer per well. Next, 160 µL of the mixture was transferred to a black microplate; 40 µL D-luciferin was added to each well. After a 2-min reaction at room temperature, luminescence at 560 nm was measured.

Transfection results for various HBPA-E in A549 and HEK293T cells (mRNA and DNA) are shown in FIGS. 6-9. The individual experiments are as follows:

For FIG. 6, polymers from Control 1 and Experiments 1, 2, 3, 5, 6, and 7 were complexed with mRNA at a polymer/mRNA mass ratio of 75:1 and assessed for mRNA transfection in A549 and HEK293T cells.

FIG. 6 shows that in A549 cells, all hyperbranched materials achieved significantly higher mRNA transfection than the commercial control LipoMM and far exceeded linear PACA-E14; HBPA-E14-3 was the highest, about 4× LipoMM. Linear PACA-E14 underperformed LipoMM. The transfection results for HEK293T mirrored those for A549.

These data indicate hyperbranched polymers markedly outperform linear analogs and even high-performance commercial reagents, with reproducibility across cell types. Given their ethanol solubility, hyperbranched polymers show strong potential for both in vitro and in vivo mRNA delivery.

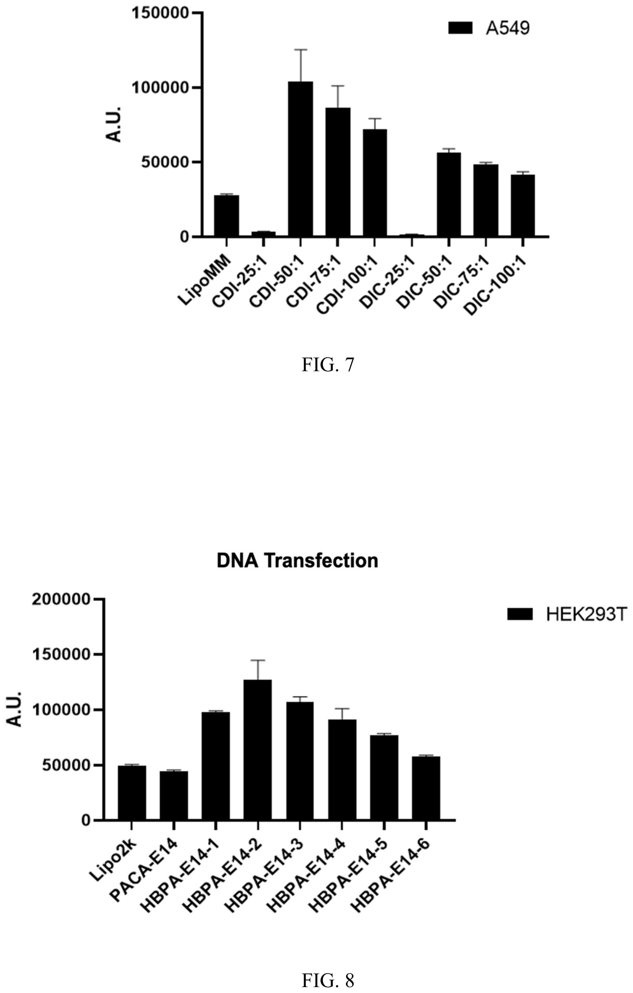

In addition, mRNA transfection efficiencies for HBPA-E14-3 prepared via two terminal-group modification methods-CDI and DIC-across different polymer-to-mRNA mass ratios were evaluated. Specifically, CDI-modified HBPA-E14-3 (Experiment 3) was complexed with mRNA at mass ratios of 25:1, 50:1, 75:1, and 100:1 and tested in A549 cells; likewise, DIC-modified HBPA-E14-3 (HBPA-E14-3-DIC, Experiment 4) was tested at the same mass ratios. The results are shown in FIG. 7.

As shown in FIG. 7, in A549 cells both CDI- and DIC-modified HBPA-E14-3 achieved transfection efficiencies significantly higher than LipoMM at polymer/mRNA mass ratios from 50:1 to 100:1, with the CDI-modified material outperforming the DIC-modified counterpart. At a 25:1 ratio, no appreciable transfection was observed. These findings indicate that both modification methods are feasible. They also suggest that at 25:1 the mRNA is not yet fully complexed, whereas complexation is achieved at 50:1 ratio or above-consistent with the particle-size results measured by DLS.

In addition, DNA transfection efficiencies of various HBPA-E materials in HEK293T cells were evaluated in this example. Specifically, polymers from Control 1 and Experiments 1, 2, 3, 5, 6, and 7 were each complexed with DNA at a polymer/DNA mass ratio of 75:1, and their transfection performance in HEK293T cells was measured. The results are shown in FIG. 8.

As shown in FIG. 8, all hyperbranched materials exhibited DNA transfection efficiencies significantly higher than the commercial control Lipofectamine 2000 (Lipo2k), and far exceeding those of the linear PACA-E14. Among them, HBPA-E14-2 achieved the highest transfection efficiency. These data indicate that hyperbranched polymer materials are also suitable for DNA transfection.

It should be understood that the HBPA-E in this example is applicable to mRNA and DNA and is expected to work with other nucleic acids, including but not limited to circRNA, saRNA, siRNA, miRNA, antisense oligonucleotides, shRNA, and small activating RNA.

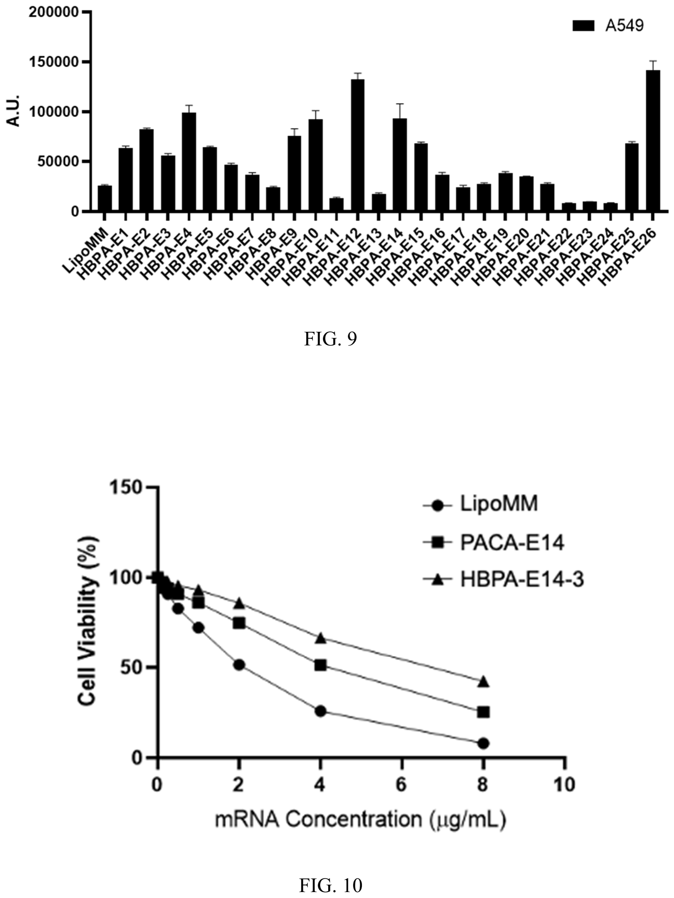

Finally, mRNA transfection efficiencies in A549 cells of HBPA-E polymers bearing 26 different terminal-group modifiers were evaluated in this example. Specifically, HBPA-E variants modified with terminal groups E1 through E26 were each complexed with mRNA at a polymer/mRNA mass ratio of 75: 1, and their transfection performance in A549 cells was measured. The results are shown in FIG. 9.

The results in FIG. 9 indicate that terminal-group chemistry influences mRNA transfection efficiency. Several terminal-group-modified HBPA-E variants showed markedly higher efficiency than LipoMM, including E2, E4, E9, E10, E12, E14, E15, E25, and E26. Except for polymers modified with E11, E13, E22, E23, and E24-which performed clearly lower than LipoMM. Variants bearing E8, E17, E18, and E21 achieved efficiencies comparable to LipoMM, while all remaining terminal-group-modified polymers outperformed LipoMM.

It should be understood that, in addition to A549 cells, the HBPA-E materials described herein are also suitable for transfection in other commonly used cell types, such as HEK293T, HeLa, U87, HUVEC, Jurkat, RAW264.7, iPSC, or MSC.

### V. Cytotoxicity Assessment

Cytotoxicity of HBPA-E/mRNA complexes was tested in A549 cells. Specifically, A549 were seeded in 96-well plates (1 × 10⁴/well). After 12 h adhesion, DMEM was refreshed and complexes of PACA-E14, HBPA-E14-3, or LipoMM with mRNA (polymer/mRNA = 50:1) were added for 24 h. Final mRNA concentration per well: 0.125, 0.25, 0.5, 1, 2, 4, and 8 µg/mL. Then 10 µL CCK-8 was added per well and absorbance at 450 nm was read. The results are shown in FIG. 10.

The results in FIG. 10 show that the LipoMM control complex exhibited marked cytotoxicity: at an mRNA concentration of 2 µg/mL, cell viability was only about 50%, and toxicity increased markedly with higher mRNA concentrations. By comparison, the linear PACA-E14 complex showed substantially reduced toxicity, though some cytotoxicity remained (cell viability about 70% at 2 µg/mL mRNA). In contrast to both, the hyperbranched HBPA-E14-3 complex demonstrated further reduced toxicity, with cell viability exceeding 80% at 2 µg/mL mRNA. These findings indicate that HBPA-E14-3 has higher biocompatibility than the commercial transfection reagent and the linear PACA-E14, suggesting stronger potential for in vivo applications.

### VI. In Vivo mRNA Delivery by HBPA-E

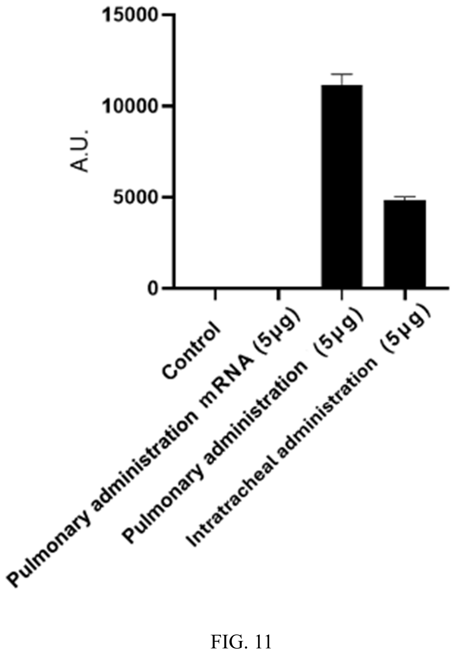

This example evaluated the in vivo mRNA transfection performance of HBPA-E 14-3. Specifically, C57 mice aged 4-6 weeks (body weight about 20 g) were purchased from the Guangdong Provincial Laboratory Animal Center and housed under SPF (specific pathogen-free) conditions. HBPA-E14-3/mRNA complexes were administered either by pulmonary delivery (using a pulmonary microsprayer inserted into the trachea; 5 µg dose) or by intratracheal instillation (5 µg dose). Luciferase expression in vivo was monitored, with normal saline as the negative control. The polymer/mRNA mass ratio in the HBPA-E14-3/mRNA complex was 50:1. Results are shown in FIG. 11.

Refer to FIG. 11, the x-axis labels denote: "Control" (saline, no mRNA), "Pulmonary delivery of mRNA (5 µg)" (naked mRNA delivered to the lung), "Pulmonary delivery (5 µg)" (HBPA-E14-3/mRNA complex delivered to the lung), and "Intratracheal instillation (5 µg)" (HBPA-E14-3/mRNA complex delivered by intratracheal instillation).

As shown in FIG. 11, no bioluminescence was detected in either the naked-mRNA pulmonary delivery group or the saline control. In contrast, the HBPA-E14-3/mRNA complex produced robust luciferase expression via both administration routes, with pulmonary delivery yielding approximately twice the transfection efficiency of intratracheal instillation. These results indicate that the hyperbranched polymer/mRNA complex enables effective in vivo mRNA delivery and holds strong promise for further in vivo applications.

### Example II

### I. Synthesis of Hyperbranched Polymer (HBPA) and Terminal-group-Modified HBPA (HBPA-E)

### (1) HBPA Synthesis

In this example, a hyperbranched polymer (HBPA) was first synthesized by copolymerizing four monomers P, S, M, and T, and the resulting HBPA was then terminal-group-modified with reagent E to afford HBPA-E. Specifically, monomer P was pentadecalactone (PDL), S was sebacic acid (SA), M was methyldiethanolamine (MDEA), and T was triethanolamine (TEA). The HBPA synthesis route is shown in FIG. 1(a). Twelve experimental runs and three controls were conducted. The four monomers P (PDL)/S (SA)/M (MDEA)/T (TEA) were charged into a round-bottom flask according to the feed molar ratios listed in Table 2, followed by immobilized lipase (CALB) at 10 wt% relative to the total mass of monomers, and finally diphenyl ether at 200 wt% relative to the total mass of monomers. After purging the reaction system with argon three times, the mixture was stirred and heated at 90 °C under 60 mbar for 24 h, then further reacted at 2.1 mbar for 48 h. Upon completion, the reaction mixture was filtered to remove the lipase. N-hexane was added to the filtrate, followed by vortexing and centrifugation; the supernatant was decanted. The precipitate was dissolved in dichloromethane, re-precipitated with n-hexane, centrifuged, and the supernatant discarded. This dissolution/precipitation procedure was repeated three times. The final precipitate was dried under vacuum for one day to obtain HBPA.

**Table 2. Polymers from different feed ratios and different degrees of terminal-group E modification**

| Serial No. | Polymer name | P/S/M/T (monomer feed molar ratio) | Amount of compound E (eq) | Relative concentration of group E |
|---|---|---|---|---|
| Control1 | HBPA-E14-2-40eq | 1: 9: 6.2: 1 | 40 | 1 |
| Experiment 1 | HBPA-E14-2-4eq | 1: 9: 6.2: 1 | 4 | 0.5 |
| Experiment 2 | HBPA-E14-2-5eq | 1: 9: 6.2: 1 | 5 | 0.6 |
| Experiment 3 | HBPA-E14-2-6eq | 1: 9: 6.2: 1 | 6 | 0.67 |
| Experiment 4 | HBPA-E14-2-7eq | 1: 9: 6.2: 1 | 7 | 0.73 |
| Control 2 | HBPA-E14-5-40eq | 1:9:3:4 | 40 | 1 |
| Experiment 5 | HBPA-E14-5-4eq | 1: 9: 3: 4 | 4 | 0.48 |
| Experiment 6 | HBPA-E14-5-5eq | 1: 9: 3: 4 | 5 | 0.55 |
| Experiment 7 | HBPA-E14-5-6eq | 1: 9: 3: 4 | 6 | 0.62 |
| Experiment 8 | HBPA-E14-5-7eq | 1: 9: 3: 4 | 7 | 0.68 |
| Control 3 | HBPA-E2-2-40eq | 1: 9: 6.2: 1 | 40 | 1 |
| Experiment 9 | HBPA-E2-2-4eq | 1: 9: 6.2: 1 | 4 | 0.52 |
| Experiment 10 | HBPA-E2-2-5eq | 1: 9: 6.2: 1 | 5 | 0.58 |
| Experiment 11 | HBPA-E2-2-6eq | 1: 9: 6.2: 1 | 6 | 0.65 |
| Experiment 12 | HBPA-E2-2-7eq | 1: 9: 6.2: 1 | 7 | 0.75 |

In Table 2, for example, "HBPA-E14-2-40eq," the "HBPA-E14-2" corresponds to the polymer name in Table 1 of Example 1, and "40eq" means that 40 equivalents of E14 were added during terminal-group E modification; the others are analogous. "HBPA-E2-2-40eq" means that E2 was used instead of E14, and 40 equivalents of E2 were added.

### (2) Terminal-group E Modification

As shown in Table 2, terminal-group modification of the polymer was carried out using terminal-group modifiers E14 and E2 with N,N'-carbonyldiimidazole (CDI) as the coupling agent. As can be seen from Table 2, this example is effectively based on "Experiment 2" and "Experiment 6" in Table 1 of Example I, with the equivalents of the terminal-group modifier E adjusted accordingly. For instance, HBPA-E14 was prepared using 40, 4, 5, 6, or 7 equivalents of E14, and HBPA-E2 was analogously prepared using E2.

Specifically, using CDI as the coupling agent (synthetic route shown in FIG. 1(b)), 250 mg of HBPA was dissolved in 5 mL of anhydrous dichloromethane (DCM) under stirring, followed by the addition of CDI (20 equivalents). After purging the reaction vessel with argon three times, the mixture was stirred at room temperature overnight. The reaction mixture was then concentrated to about 3 mL, three volumes of diethyl ether were added, vortexed, and centrifuged, and the resulting precipitate was discarded. The supernatant was rotary evaporated to dryness under reduced pressure, the residue was redissolved in anhydrous DCM, and-per Table 2-different equivalents of E14 were added under stirring (specifically 40, 4, 5, 6, or 7 equivalents). The reaction was proceeded at room temperature for 24 hours. Upon completion, an equal volume of deionized water was added to the reaction mixture; the phases were vortexed, centrifuged, and allowed to separate, and the upper aqueous phase was removed. This washing with an equal volume of deionized water was repeated five times. Three volumes of n-hexane were then added to the lower DCM phase, followed by vortexing and centrifugation. The resulting solid was collected and dried under vacuum for one day to afford HBPA-E14.

Using methods similar to Experiments 1-4, this example further performed terminal-group modification of HBPA with terminal-group modifiers other than E14 and E2-namely the remaining twenty-four modifiers among E1-E26 (excluding E14 and E2)-to synthesize additional HBPA-E materials with varying degrees of terminal-group substitution. The set of twenty-six modifiers E1-E26 is the same as in Example 1.

### II. Structural Characterization of HBPA-E

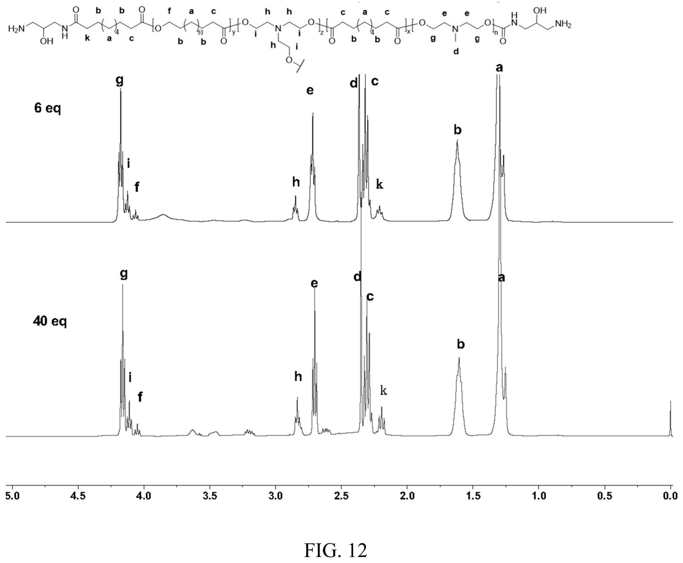

The molecular structures of HBPA-E samples with different degrees of terminal-group substitution were characterized by 1H NMR. Representative results are shown in FIG. 12. FIG. 12 presents the 1H NMR spectra (solvent: CDCl₃) of HBPA samples from Table 2 after different degrees of terminal-group E14 modification-namely HBPA-E14-2-40eq (Control 1) and HBPA-E14-2-6eq (Experiment 3). In the spectra, the characteristic peaks of the PDL unit appear at f (δ ≈ 4.08 ppm), the SA unit at b (δ ≈ 1.60 ppm), the MDEA unit at g (δ ≈ 4.20 ppm), and the TEA unit at h (δ ≈ 2.85 ppm). The peaks at k (δ ≈ 2.22 ppm) evidence successful modification of terminal-group E. By comparing the relative intensities of these diagnostic peaks, the degree of E14 substitution can be assessed. Under comparable b peak intensities, the k peak of HBPA-E14-2-6eq is markedly weaker than that of HBPA-E14-2-40eq, indicating a significantly lower E14 substitution level in the 6eq sample. These data demonstrate that the final degree of E14 modification in HBPA-E 14 can be tuned by controlling the feed amount of E14 during the reaction.

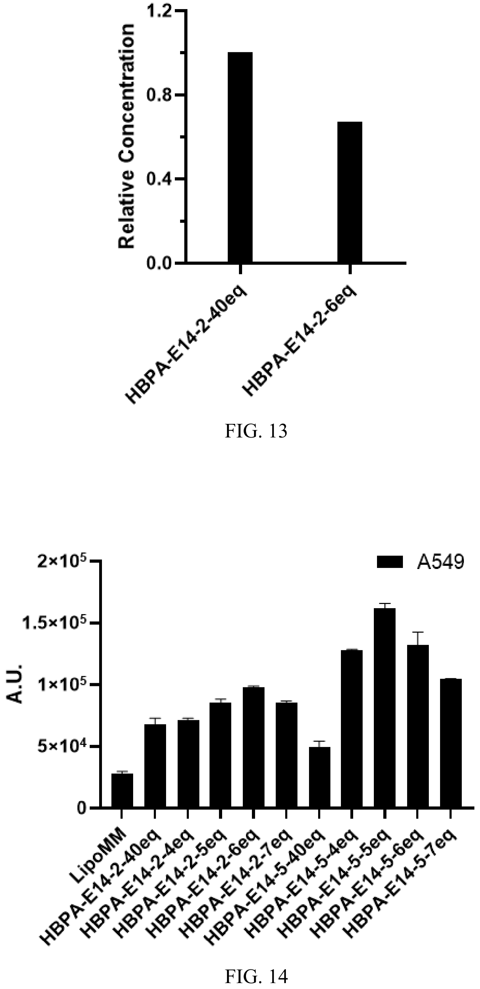

Further, in this example the E14 terminal-group content of HBPA-E14-2-40eq and HBPA-E 14-2-6eq was quantified by the ninhydrin method. The specific procedure was as follows: (1) Preparation of ninhydrin chromogenic reagent: Weigh 200 mg of ninhydrin, dissolve it in 9 mL of ethanol, then dilute with ethanol to 10 mL to obtain a 20 mg/mL ninhydrin ethanol solution. (2) Sample determination: Weigh about 10 mg of each sample and prepare 5 mg/mL ethanol solutions. Take 100 µL of each sample solution and mix with 200 µL of the ninhydrin ethanol solution, mix well, and place on a shaker at 60 °C with shaking for 10 min. After cooling to room temperature, take 200 µL of each reaction solution and measure the absorbance at 570 nm. The absorbance values are then converted into the relative concentration of E14 terminal groups. The results are shown in FIG. 13. Setting the concentration of E14 terminal groups in HBPA-E14-2-40eq to 1, under the same polymer concentration, the relative concentration of E14 terminal groups in HBPA-E14-2-6eq is 0.67, indicating that its E14 terminal-group content is 0.67 times that of HBPA-E14-2-40eq. This quantitatively demonstrates that by controlling the amount of E14 added to the reaction, the degree of E14 modification in the final HBPA-E14 can be tuned. That is, using 40 eq of E14 yields HBPA-E14 in which almost all terminal groups are modified with E14, whereas using 4-7 eq of E14 yields HBPA-E14 with only partial terminal-group modification, with relative E14 terminal-group concentrations of 0.5, 0.6, 0.67, and 0.73, respectively.

### III. In vitro mRNA transfection efficiency of HBPA-E in cells

After preparing polymer materials HBPA-E with different degrees of terminal-group modification, these polymers were complexed with Firefly Luciferase mRNA (N1-Me-Pseudo UTP, Nanjing Vazyme Biotech Co., Ltd.) at a polymer/mRNA mass ratio of 50:1. Specifically, HBPA-E polymers were first dissolved in ethanol to prepare 25 mg/mL polymer solutions. The polymer solutions were then added into an appropriate volume of NaAc buffer (pH 4.9) with vortex mixing. At the same time, a NaAc solution (pH 4.9) containing 20 µg/mL mRNA was prepared. Equal volumes of the polymer solution and the mRNA solution were mixed by vortexing to obtain the final polymer-mRNA complex solutions.

The mRNA transfection efficiency of HBPA-E/mRNA complexes was evaluated in an A549 cell model. The specific transfection procedure was as follows: A549 cells (ATCC) were seeded in 48-well plates at 2.5 × 10⁴ cells/well. After 12 h of culture at 37 °C in DMEM containing 5% CO₂ to allow cell attachment, the medium was replaced with fresh DMEM (250 µL/well). Test samples and the positive control were then added and cells were incubated for 24 h, with the sample amount calculated to give a final mRNA concentration of 2 µg/mL per well. Lipofectamine MessengerMAX (LipoMM, Thermo Fisher) was used as the positive control for mRNA transfection.

The procedure for cell lysis and luminescence measurement after transfection was as follows: 24 h after mRNA transfection, the culture medium in each well was aspirated, and the plate was placed at -80 °C for 10 min. The plate was then equilibrated at 4 °C, 40 µL of lysis buffer was added to each well to cover the bottom, and the plate was left to stand for 5 min. Then, 280 µL of assay buffer was added to each well, and 160 µL of the resulting mixture was transferred to a black microplate. Finally, 40 µL of D-luciferin was added to each well using a multichannel pipette. After reacting at room temperature for 2 min, luminescence at 560 nm was measured using a microplate reader.

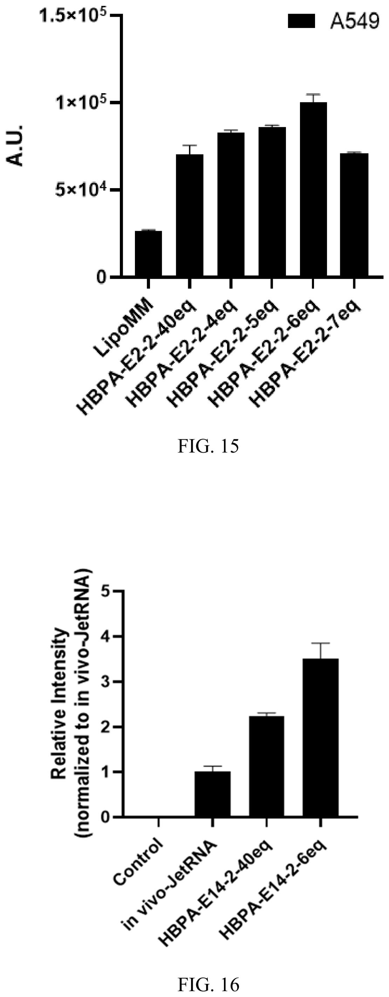

The mRNA transfection efficiencies of different HBPA-E polymers in A549 cells are shown in FIGS. 14 and 15. The specific experiments were as follows:
In this example, E14-modified polymers (controls 1 and 2, experiments 1-8) were complexed with mRNA at a polymer/mRNA mass ratio of 50:1, and their mRNA transfection efficiencies in A549 cells were determined, as shown in FIG. 14.

The results in FIG. 14 show that in A549 cells, all E14-modified HBPA-E polymers exhibited significantly higher mRNA transfection efficiency than the commercial control LipoMM. For the different HBPA backbones, polymers with E14 modification levels of 4-7 eq all showed higher transfection efficiency than those with 40 eq E14. Among them, for HBPA-E14-2, HBPA-E14-2-6eq gave the highest transfection efficiency, approximately 1.5 times that of HBPA-E14-2-40eq; for HBPA-E14-5, HBPA-E14-5-5eq showed the highest transfection efficiency, more than 3 times that of HBPA-E14-5-40eq. This indicates that for hyperbranched polymers, HBPA-E with a lower degree of terminal-group modification can achieve higher delivery efficiency than those with maximal terminal-group modification. In other words, when the relative concentration of E14 terminal groups is 0.48-0.75, such that only part of the terminal groups are modified, higher transfection efficiency can be obtained.

In addition, this example also evaluated E2-modified polymers (control 3, experiments 9-12) complexed with mRNA at a polymer/mRNA mass ratio of 50:1, and their mRNA transfection efficiencies in A549 cells, as shown in FIG. 15.

The results in FIG. 15 show that in A549 cells, similar to the case of E14 modification, all E2-modified HBPA-E polymers displayed significantly higher mRNA transfection efficiency than the commercial control LipoMM. For different HBPA backbones, polymers with E2 modification levels of 4-7 eq all exhibited higher transfection efficiency than those with 40 eq E2. Among them, HBPA-E2-2-6eq gave the highest transfection efficiency, approximately 1.5 times that of HBPA-E2-2-40eq. This indicates that modifying only a portion of the terminal groups can enhance transfection efficiency, and this applies to different terminal-group compounds.

It can be understood that the technical effect of improving transfection efficiency mainly depends on the specific charge content and spatial distribution generated by partial terminal-group modification. Therefore, it can be expected that, in addition to E2 and E14, other terminal-group-modified compounds E1-E26 will also be able to improve transfection efficiency to varying degrees when only partial terminal-group modification is performed.

### IV. In vivo evaluation of mRNA delivery by HBPA-E

In this example, the in vivo mRNA delivery performance of HBPA-E 14-2-40eq and HBPA-E14-2-6eq was compared. The specific method was as follows: C57 mice aged 4-6 weeks (body weight ~20 g) were purchased from the Guangdong Provincial Experimental Animal Center and housed under specific pathogen-free (SPF) conditions. The two HBPA-E14-2/mRNA complexes were administered to mice via pulmonary delivery (intratracheal administration using a lung microsprayer, 5 µg dose), and the in vivo luciferase expression was evaluated. Physiological saline was used as the blank control, and the commercial reagent in vivo-JetRNA for in vivo delivery was used as the positive control. The results are shown in FIG. 16. In the HBPA-E14-2/mRNA complexes, the mass ratio of HBPA-E 14-2 to mRNA was 50:1.

In FIG. 16, the x-axis label "Control" denotes the blank group; "in vivo-JetRNA" denotes pulmonary administration of in vivo-JetRNA/mRNA; "HBPA-E14-2-40eq" denotes pulmonary administration of the HBPA-E14-2-40eq/mRNA complex; and "HBPA-E14-2-6eq" denotes pulmonary administration of the HBPA-E 14-2-6eq/mRNA complex. The y-axis values are normalized to the result of the in vivo-JetRNA group.

The results in FIG. 16 show that no luminescence was detected in the blank group. Compared with the commercial in vivo-JetRNA/mRNA group, both HBPA-E14-2/mRNA complexes exhibited higher luciferase expression, with the delivery efficiency of HBPA-E14-2-6eq being 1.5 times that of the HBPA-E14-2-40eq group. This is consistent with the cell transfection results and demonstrates that HBPA-E with a lower degree of terminal-group modification can achieve higher delivery efficiency than those with maximal terminal-group modification.

The above contents are detailed descriptions of the present application in combination with specific embodiments, and it cannot be determined that the specific embodiments of the present application is limited to these descriptions. For an ordinary person skilled in the field to which the present application belongs, several simple deductions or substitutions can be made without departing from the concept of the present application.

## Claims

1. A cationic polyester, **characterized in that**: the cationic polyester is a hyperbranched polymer formed by polymerizing three monomers, monomer P, monomer S and monomer M, or two monomers, monomer P and monomer S, with monomer T, the end group of the hyperbranched polymer is modified with a group E, and the relative concentration of the end group E relative to the end group E of the hyperbranched polymer with complete end group modification is 0.48-0.75;
wherein, monomer P is a lactone;
monomer S is an organic acid containing two or more terminal carboxyl groups;
monomer M is a compound containing two hydroxyl groups and a secondary or tertiary amine;
monomer T is a polyol containing three or more hydroxyl groups; and
the terminal-modifying compound E contains at least one primary, secondary, or tertiary amine.

2. The cationic polyester according to claim 1, **characterized in that**: the monomer P is a lactone having an aliphatic chain length of 6 to 35;
preferably, the monomer P is at least one of caprolactone, dodecalactone, pentadecalactone, and hexadecalactone.

3. The cationic polyester according to claim 1, **characterized in that**: the monomer S is an organic acid having a carbon chain length of 3 to 18;
preferably, the monomer S is at least one of adipic acid, sebacic acid, and 1,2,3-propanetricarboxylic acid.

4. The cationic polyester according to claim 1, **characterized in that**: the monomer M is a compound having a carbon chain length of 4 to 36;
preferably, the monomer M is at least one of diethanolamine, methyldiethanolamine, and ethyldiethanolamine.

5. The cationic polyester according to claim 1, **characterized in that**: the monomer T is a compound having a carbon chain length of 4 to 54;
preferably, the monomer T is at least one of trimethylolpropane, 3-(hydroxymethyl)-1,5-pentanediol, triethanolamine, and N,N,N',N'-tetrakis(2-hydroxyethyl)ethylenediamine.

6. The cationic polyester according to claim 1, **characterized in that**: the end-group modifying compound E that provides group E is at least one of E1 to E26
| | | | | | |
|---|---|---|---|---|---|
| E1 | 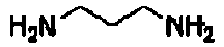 | E10 | 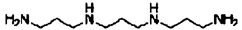 | E18 | 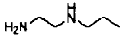 |
| E2 | 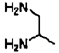 | E11 | 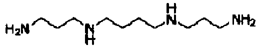 | E19 | 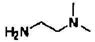 |
| E3 | 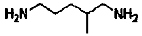 | E12 | 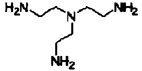 | E20 | 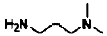 |
| E4 | 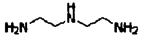 | | | E21 | 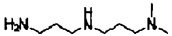 |
| E5 | 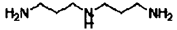 | E13 | 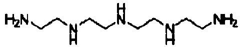 | E22 | 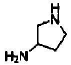 |
| E8 | 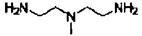 | E14 | 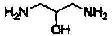 | E23 | 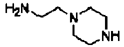 |
| E7 | 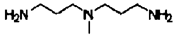 | E15 | 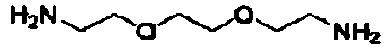 | E24 | 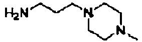 |
| E8 | 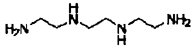 | E18 | 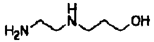 | E25 | 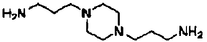 |
| E9 : | 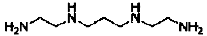 | E17 | 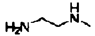 | E26 | 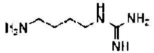 |
preferably, the end-group modifying compound E that provides group E is at least one of E1-E10, E12, E14-E21, E25, and E26;
more preferably, the end-group modifying compound E that provides group E is at least one of E1, E2, E3, E4, E5, E6, E9, E10, E12, E14, E15, E25, and E26.

7. The cationic polyester according to any one of claims 1 to 6, **characterized in that**: the cationic polyester is a hyperbranched polymer of the structure shown in Formula I; 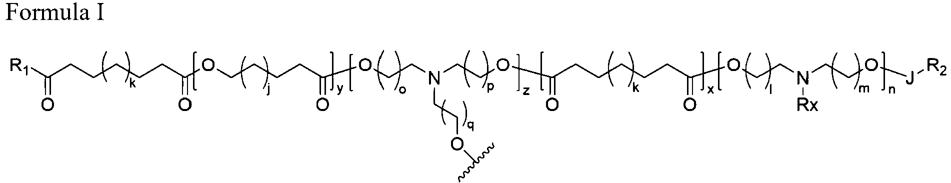
wherein, x, y, z are independent integers from 1 to 200,
n is an integer from 0 to 200,
j and k are integers from 0 to 30,
l, m, o, p, and q are independent integers from 1 to 20,
Rₓ is hydrogen; a substituted or unsubstituted alkyl group containing 1 to 18 carbon atoms; a substituted or unsubstituted aryl with at least one phenyl ring; a substituted or unsubstituted heterocycle with at least one hetero ring; or a substituted or unsubstituted alkoxy group containing 1 to 18 carbon atoms and at least one oxygen atom;
R₁ is fully modified by group E provided by the end-group modifier E, or partially modified by group E provided by the end-group modifier E, with any remainder being hydroxyl.
J is hydrogen or carbonyl; when J is hydrogen, no R₂ is present; when J is carbonyl, R₂ is group E provided by the end-group modifier E;
in Formula I, cut lines indicate branching; the first monomer connected at the branch is P or S, and subsequent monomers form the hyperbranched structure.

8. The cationic polyester according to any one of claims 1 to 6, **characterized in that**: the number-average molecular weight is 1-30 k, preferably 2-20 k.

9. Use of the cationic polyester according to any one of claims 1 to 8 in nucleic acid drug delivery.

10. The use according to claim 9, **characterized in that**: the nucleic acid drug includes but is not limited to mRNA, circRNA, saRNA, siRNA, miRNA, antisense oligonucleotides, shRNA, small activating RNA, and DNA.

11. The use according to claim 9 or 10, **characterized in that**: the nucleic acid drug delivery comprises encapsulating the nucleic acid drug with the cationic polyester and delivering it into cells;
preferably, the types of cells include but are not limited to HEK293T, A549, HeLa, U87, HUVEC, Jurkat, RAW264.7, iPSC, and MSC.

12. A nucleic acid delivery particle, **characterized in that**: comprising an encapsulating material and a nucleic acid encapsulated in the encapsulating material;
the encapsulating material comprises the cationic polyester according to any one of claims 1 to 8; and
the nucleic acid is at least one of mRNA, circRNA, saRNA, siRNA, miRNA, antisense oligonucleotides, shRNA, small activating RNA, and DNA.

13. The nucleic acid delivery particle according to claim 12, **characterized in that**: the particle size of the nucleic acid delivery particle is 30-500 nm.

14. A kit for nucleic acid drug delivery, **characterized in that**: comprising at least one of the following components:
(a) the cationic polyester according to any one of claims 1 to 8;
(b) the nucleic acid delivery particle according to claim 12 or 13.

15. A method for improving transfection efficiency in nucleic acid drug delivery, **characterized in that**: comprising encapsulating a nucleic acid drug with the cationic polyester according to any one of claims 1 to 8, or with an encapsulating material comprising the cationic polyester according to any one of claims 1 to 8, to form nucleic acid delivery particle; and transfecting cells with the nucleic acid drug in the nucleic acid delivery particle.

16. Use of the cationic polyester according to any one of claims 1 to 8, the nucleic acid delivery particle according to claim 12 or 13, or the kit according to claim 14, for the treatment of diseases based on nucleic acid delivery.

17. A method of administering a nucleic acid drug in vivo, **characterized in that**: encapsulating the nucleic acid drug with the cationic polyester according to any one of claims 1 to 8, or with an encapsulating material comprising the cationic polyester according to any one of claims 1 to 8, to form nucleic acid delivery particle, and delivering the nucleic acid delivery particle in vivo; or, directly delivering the nucleic acid drug with nucleic acid delivery particle according to claim 12 or 13 in vivo.
